# EUROPEAN PATENT SPECIFICATION

(11) **EP 0 112 332 B1**
(45) Date of publication and mention of the grant of the patent: **16.06.1993**
(21) Application number: 82902413.2
(22) Date of filing: 30.06.1982
(51) Int. Cl.: A61K 31/70, C07H 15/20

(54) **VITAMIN D GLYCOSIDES**
VITAMIN D GLYCOSIDE
GLYCOSIDES DE LA VITAMINE D

(43) Date of publication of application: 04.07.1984
(73) Proprietor: THE GENERAL HOSPITAL CORPORATION, Boston, MA 02114 (US)
(72) Inventor: HOLICK, Sally Ann, Sudbury, MA 01776 (US); HOLICK, Michael Francis, Subbury, MA 01776 (US)
(74) Representative: Tiedtke, Harro, Dipl.-Ing.
(86) International application number: US8200882
(87) International publication number: WO8400107

(56) References cited:
- US-A- 4 188 345
- US-A- 4 292 250
- CHEMICAL ABSTRACTS, vol. 84, no. 5, 2nd February 1976, page 497, no. 31373n, Columbus, Ohio, US; & JP - A - 75 101 351 (K. SHIRAI) 11-08-1975
- N, Life Science, Volume 18, No. 10, Issued 1976, Mark R. Haussler, et al., "1,25 -Dihydroxyvitamin D3-Glycoside", pp. 1049-1056
- N, Nature, Volume 268, No. 5618, Issued 1977 Mark R. Hughes et al., "Presence of 1,25-dihydroxyvitamin D3 glycoside in the Calcinogenic Plant Cestrum diurnum" pp. 347-349
- N, Journal of Clinical Investigation, Volume 66, Issued 1980, Sreeramulu Nagubandi et al., "Role of Vitamin D Glucosiduronate in Calcium Homeostasispp. 1274-1280

## Description

The present invention relates to water-soluble synthetic glycosides of vitamin D, and their use in the regulation of calcium metabolism.

### Background Art

Vitamin D₃ deficiency, or disturbances in the metabolism of vitamin D₃ cause such diseases as ricketts, renal osteodystrophy and related bone diseases, as well as, generally, hypo- and hyper-calcemic states. vitamin D₃ and its metabolites are therefore crucial in maintaining normal development of bone structure by regulating blood calcium levels.

vitamin D₃ is rapidly converted to 25-OH-D₃ in the liver. In response to hypocalcemia, 25-OH-D₃, the major circulating metabolite of the vitamin, undergoes further metabolism in the kidney to 1α, 25-(OH)₂D₃. 1α, 25-(OH)₂D₃ acts more rapidly than either D₃ or 25-OH-D₃. Additionally, the dihydroxy form of the vitamin is 5-10 times more potent than D₃ and about 2-5 times more potent than the monohydroxy form of the vitamin, in vivo, provided it is dosed parenteraly and daily (Napoli, J.L. and Deluca, H.F., "Blood Calcium Regulators" and references cited therein in: Burger's Medicinal Chemistry, 4th Ed., part II, edited by Manfred Wolf, Wiley-Interscience, 1979, pp-725-739.)

vitamin D₂, vitamin D₃ or their metabolites which are hydroxylated at positions 1; 1, 25; 1,24, 25; 24,25; 25,26; or 1,25,26 are water-insoluble compounds. When a drug is relatively insoluble in a aqueous environment or in the gastrointestinal lumen, post-administration dissolution may become the rate-limiting step in drug absorption. On the other hand, with water-soluble drugs, dissolution occurs rapidly and thus facilitates transport through the blood and to the site of activity. It would therefore be desirable to provide a form of vitamin D (D₃ or D₂) which is hydrophilic and/or water-soluble, yet preserves the normal biological properties of the water-insoluble drug.

The extracts from the leaves of a South American plant, Solanum malacoxylon (hereinafter "S.m."), have been demonstrated to contain a water-soluble principle which is different than 1,25(OH)₂D₃ and which, upon treatment with glycosidase enzymes yields 1,25(OH)₂D₃, plus a water-soluble unidentified fragment. (See, for example, Haussler, M.R., et al, Life Sciences, Volume 18: 1049-1056 (1976); Wasserman R.H. et al, Science 194: 853-855 (1976); Napoli, J.L. et al, The Journal of Biological Chemistry, 252: 2580-2583 (1977)).

A very similar water-soluble principle, which upon treatment with glycosidases also yields 1,25 dihydroxy vitamin D₃, is found in the plant Cestrum diurnum (hereinafter "C.d."; Hughes, M.R., et al, Nature, 268: 347-349 (1977)). The water soluble extracts from S.m. or C.d. have biological activity which is similar to that of 1α, 25-dihydroxy vitamin D₃.

The only evidence existing to date concerning the structure of the water-soluble fragment released during glycosidase treatment of the water-soluble principles from these plants is indefinite. The authors of the aforementioned publications have concluded that the structure is probably a glycoside, on the basis of enzymatic evidence, the water-solubility, and the use of chemical detection reagents (Peterlik, N. and Wasserman, R.H. FEBS Lett. 56: 16-19, (1973)). Humphreys (Nature (London) New Biology 246: 155 (1973)), however, has cast some doubt on this conclusion since he demonstrated that the Molisch carbohydrate test was negative for the principle.

Since it is known that the molecular weight of the water-soluble vitamin D₃-containing principle, prior to enzymatic release, is considerably greater than 1000 (Humphreys, D.J., Nature (London) New Biology 246: 155 (1973)), the molecular weight of the water-soluble conjugated fragment released by enzymatic hydrolysis can be calculated to be considerably greater 584, the molecular weight of dihydroxy vitamin D₃ being 416. Thus if the water-soluble fragment released by enzymatic hydrolysis were in fact a glycoside, it would contain more than 3 glycosidic (glycopyranosyl or glycofuranosyl) units.

Moreover, the results of enzymatic release are fully consistent with a wide variety of structures. For example, Haussler, M.R., et al, Life Sciences 18: 1049-1056 (1976) disclose the use of mixed glycosidases derived from Charonia lampus to hydrolyze the water-soluble principle. This enzyme is really a mixture of enzymes as follows (Miles Laboratories, 1977 Catalog): β- glucosidase (11 units), α- mannosidase (33 units), β- mannosidase (5.2 units), α- glucosidase, β- glucosidase (4.8 units), β- galactosidase (44 units), α- galactosidase (26 units), α- fucosidase (24 units), β- xylosidase (8.2 units), β- N-acetylglucosaminidase (210 units), α- N-acetylgalactosaminidase (41 units), and β- N-acetylgalactosaminidase (25 units) Peterlik, M., et al (Biochemical and Biophysical Research Communications, 70: 797-804 (1976)) in their study of S.m. extract with β-glucosidase (almond) from Sigma Chemical Company utilized an enzyme that also contained β- D-galactosidase, and α- D-mannosidase activities (Sigma Chemical Company, February 1981 Catalog; see also Schwartz, J., et al, Archives of Biochemistry and Biophysics, 137: 122-127 (1970)).

In sum, the results observed by these authors are consistent with a wide range of structures, none of which have been well characterized but which, even if proven to be glycosides, contain at least more than 3 glycosidic units per vitamin D unit.

A need, therefore, continues to exist for a well-defined, well-characterized water-soluble form of vitamin D, which will be hypocalcemically active and maintain calcium and phosphorus homeostasis.

### Disclosure of the Invention

It is therefore an object of the invention to provide well-characterized, well-defined synthetic, water soluble forms of vitamin D₃, vitamin D₂, and hydroxylated metabolites thereof.

It is another object of the invention to provide water-soluble forms of the aforementioned vitamins D which are hypocalcemically active, and which are active in maintaining calcium and phosphorous homeostasis in the animal body.

Still another object of the invention is to provide a pharmaceutical composition containing the aforementioned vitamins.

These and other objects of the invention, as will hereinafter become more readily apparent, have been attained by providing:

A synthetic compound which is biologically active in maintaining calcium and phosphorous homeostasis in animals, of the formula (I) 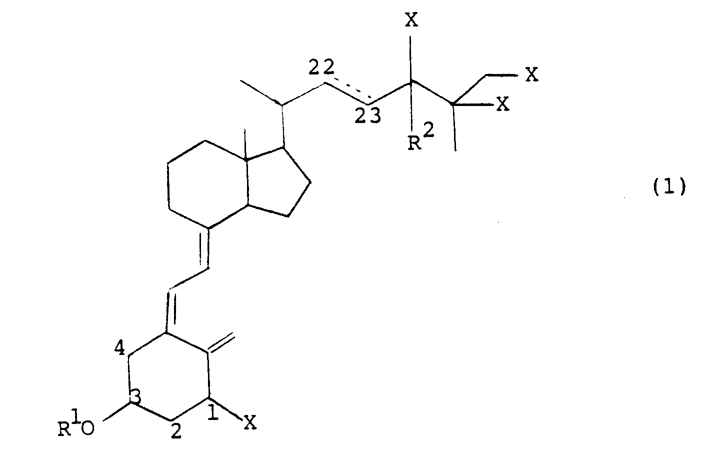
wherein the bond between carbons C-22 and C-23 is single or double; R² is hydrogen, -CH₃ or -CH₂CH₃;
wherein X is selected from hydrogen and -OR¹, wherein R¹ is a glycosidic residue selected from glucose, mannose, galactose, gulose, allose, altrose, idose, talose, fructose, arabinose, xylose, sucrose, cellobiose, maltose, lactose, trehalose, gentiobiose, meliobiose, raffinose, gentianose, N-acetyl-D-galactosamine, N-acetyl-D-glucosamine, N-acetyl-D-mannosamine, N-acetylneuraminic acid, D-glucosamine, lyxosylamine and D-galactosamine.

### Best Mode for Carrying Out the Invention

The present invention provides for the first time well-defined and substantially pure characterized synthetic, water-soluble forms of vitamins D₃ and D₂, as well as hydroxylated derivatives of these vitamins. The compounds of the present invention may in many instances be crystalline. They represent a distinct advance over the partially purified, poorly characterized presumed "glycoside" of 1α, 25-dihydroxy vitamin D₃ of the prior art.

By glycosidic units are meant glycopyranosyl or glycofuranosyl, as well as their amino sugar derivatives. The residues may be homopolymers, random, or alternating or block copolymers thereof. The glycosidic units have free hydroxy groups, or hydroxy groups acylated with a group 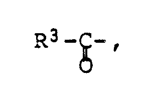
wherein R³ is hydrogen, lower alkyl, aryl or aralkyl. Preferably R³ is C₁-C₆ alkyl, most preferably acetyl or propionyl, phenyl, nitrophenyl, halophenyl, lower alkyl-substituted phenyl, lower alkoxy-substituted phenyl and the like; or benzyl, nitrobenzyl, halobenzyl, lower-alkyl-substituted benzyl and lower alkoxy-substituted benzyl

When the compounds of formula (I) have a double bond at position C-22, they are derivatives of vitamin D₂, whereas if the bond at that position is single, and there is a lack of C₂₄ alkyl they are derivatives of vitamin D₃. The latter are preferred.

The compounds of the invention contain at least one glycosidic residue at positions 1, 3, 24, 25 or 26. They may, however contain more than one, and up to five such glycosidic residues simultaneously.

Preferred are those compounds derived from vitamins D₃ or D₂; 1-hydroxy-vitamins D₃ or D₂; 1,25-dihydroxy-vitamins D₃ or D₂; 25-dihydroxy-vitamins D₃ or D₂; 25,26-dihydroxy-vitamins D₃ or D₂; 1,24,25-trihydroxy-vitamins D₃ or D₂ and 1,25,26-trihydroxy-vitamins D₃ or D₂. Most preferred among these are vitamins D₃ or D₂; 1-hydroxy-vitamins D₃ or D₂; and 1-25-dihydroxy-vitamins D₃ or D₂.

In the case of multihydroxylated forms of the vitamins (e.g., 1,25-dihydroxy-vitamin D₃ has three hydroxy groups, at positions 1, 3, and 25), the preferred compounds of the invention are those wherein less than all of the multiple hydroxy groups are glycosilated, most preferably those where only one of the multiple hydroxy groups is glycosilated.

The glycosides can comprise up to 20 glycosidic units. Preferred, however, are those having less than 10, mose preferred, those having 3 or less than 3 glycosidic units. Specific examples are those containing 1 or 2 glycosidic units in the glycoside residue.

The glycopyranose or glycofuranose rings or amino derivatives thereof may be fully or partially acylated or completely deacylated. The completely or partially acylated glycosides are useful as defined intermediates for the synthesis of the deacylated materials.

Among the possible glycopyranosyl structures are glucose, mannose, galactose, gulose, allose, altrose, idose, or talose. Among the furanosyl structures, the preferred ones are those derived from fructose, arabinose, cellobiose, maltose, lactose, trehalose, gentiobiose, and melibiose. Among the triglycosides, the preferred ones are those derived from fructose, arabinose or xylose. Among preferred diglycosides are sucrose, cellobiose, maltose, lactose, trehalose, gentiobiose, and melibiose. Among the triglycosides, the preferred ones may be raffinose or gentianose. Among the amino derivatives are N-acetyl-D-galactosamine, N-acetyl-D-glucosamine, N-acetyl-D-mannosamine, N-acetylneuraminic acid, D-glucosamine, lyxosylamine and D-galactosamine.

When more than one glycosidic unit is present on a single hydroxy group (i.e., di or polyglycosidic residues), the individual glycosidic rings may be bonded by 1-1, 1-2, 1-3, 1-4, 1-5, or 1-6 bonds, most preferably 1-2, 1-4, and 1-6. The linkages between individual glycosidic rings may be α or β.

The configuration of the oxygen linkage of a hydroxy group, or glycosidic residue attached to the vitamin D₃ or D₂ molecule may be either α (out of the plane of the paper) or β (into the plane of the paper). It is preferred if the configuration of the 3-hydroxy or glycosidoxy group at C-3 be β , and that, independently or simultaneously the configuration of the hydroxy or glycosidoxy at C-1 be α . It is also preferred that the configuration around C-24 be R. When, at C-24, X=H and R²=-CH₃ or -CH₂CH₃, the configuration at C-24 is preferably S.

Specific Examples of compounds of the invention are:
vitamin D₃, 3β-(β- D-glucopyranoside);
vitamin D₃, 3β-(β- D-fructofuranoside);
vitamin D₃, 3β-(β- cellobioside);
vitamin D₃, 3β-(β- maltoside);
vitamin D₃, 3β-(β- lactoside);
vitamin D₃, 3β-(β- trehaloside);
vitamin D₃, 3β- raffinoside;
vitamin D₃, 3β- gentiobioside;
1α- hydroxy-vitamin D₃, 3β-(β- D-glucopyranoside);
1α- hydroxy-vitamin D₃, 3β-(β-D-fructofuranoside);
1α- hydroxy-vitamin D₃, 3β-(β- cellobioside);
1α- hydroxy- 3β-(β- maltosyl) vitamin D₃;
1α- hydroxy- 3β- raffinosyl-vitamin D₃;
1α- hydroxy- 3β- gentiobiosyl-vitamin D₃;
1α-(β- D-glucopyranosyl)-vitamin D₃;
1α-(β- D-fructofuranosyl)-vitamin D₃;
1α-(β- cellobiosyl)-vitamin D₃;
1α-(β- maltosyl)-vitamin D₃;
1α-(β- lactosyl)-vitamin D₃
1α-(β- trehalosyl)-vitamin D₃;
1α- raffinosyl-vitamin D₃;
1α- gentiobiosyl-vitamin D₃;
1α, 25-dihydroxy-vitamin D₃, 3β-(β- D-fructofuranoside)
1α, 25-dihydroxy-vitamin D₃, 3β-(β- D-glucopyranoside);
1α-(β- D-glycopyranosyl)-25-hydroxy-vitamin D₃;
1α-(β- D-fructofuranosyl)-25-hydroxy-vitamin D₃;
1α- hydroxy-25- (β- D-fructofuranosyl)-vitamin D₃;
1α- hydroxy, 25- (β- cellobiosyl)-vitamin D₃;
1α- hydroxy, 25- (β- maltosyl)-vitamin D₃;
1α- hydroxy, 25- (β- lactosyl)-vitamin D₃;
1α- hydroxy, 25- (β- trehalosyl)-vitamin D₃;
1α- hydroxy, 25-raffinosyl-vitamin D₃;
1α- hydroxy, 25-gentiobisyl-vitamin D₃.

All of the aforementioned derivatives can also be prepared with vitamin D₂.

The glycosidic derivatives of vitamins D of the present invention can be prepared by standard synthetic methods well known to those skilled in the art. These methods depend on whether the starting vitamin D₃ or vitamin D₂ contains one or more hydroxy groups. When the vitamin contains only one hydroxy group, the syntheses are straightforward, since the monohydroxylated vitamin (hydroxylated at position 3) is treated with silver carbonate in a refluxing solution of an inert nonpolar solvent such as benzene or toluene, to which is added fully acylated glycoside or fully acylated straight or branched chain glycosidic polymer, either of these containing an appropriate leaving group (L.G.) at position C-1' of the terminal ring (or on the single ring, as called for). Condensation occurs according to the following reaction, indicated here for a single glycoside for purpose of illustration only: 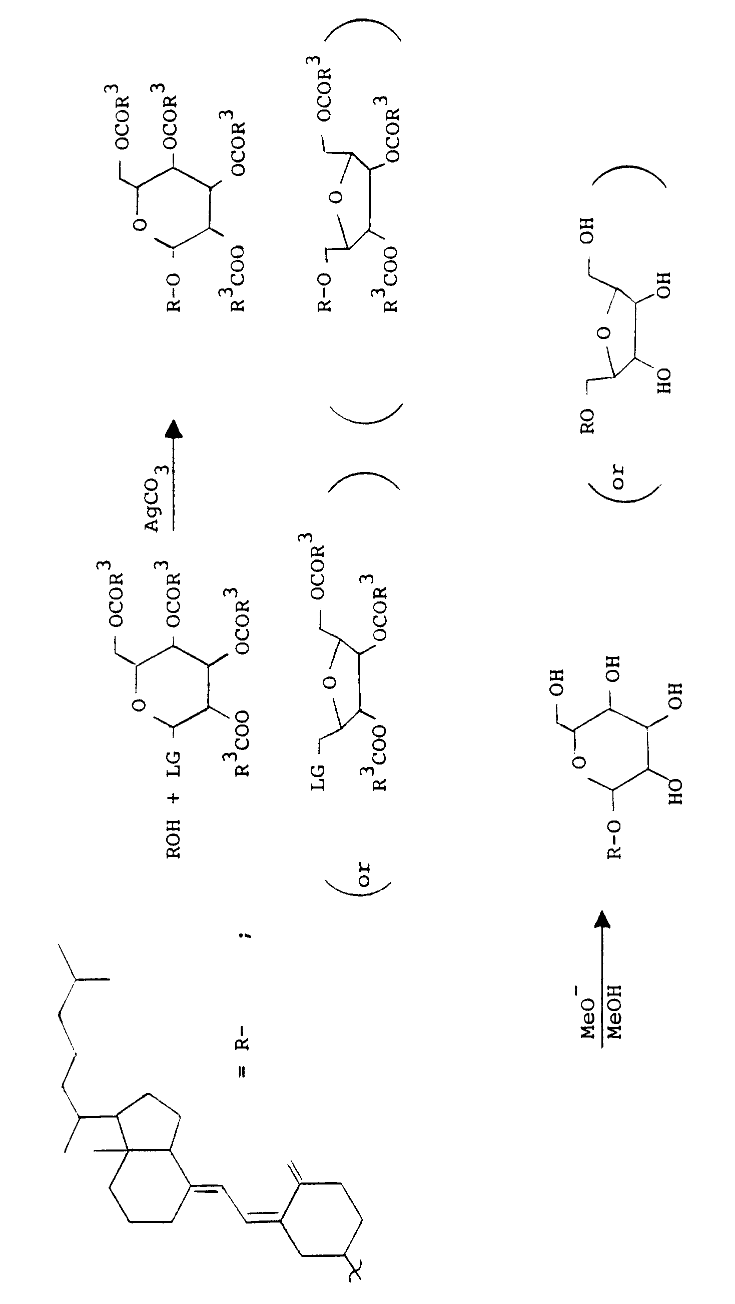
In this reaction sequence, R³ is as defined previously, LG is a common leaving group such as bromine, chlorine, iodine and p-toluenesulfonyl, capable of being replaced in a bimolecular nucleophilic substitution reaction.

When the vitamin D₃ or D₂ is reacted with a glycosidic polymer, one or more of the OCOR³ groups in the glycopyranoside or glycofuranoside rings is replaced by a fully acylated glycosidic unit, with the proviso that the total number of glycosidic units not exeed 20.

The reaction is carried out at from room temperature to refluxing conditions for a period of 1-10 hours, and is thereafter cooled and filtered to remove the silver salt. The filtrate is dried and the inert solvent is evaporated. The resulting product can be purified by any of the standard modern purification methods such as high performance liquid chromatography, silicic acid chromatography and thin layer preparative chromatography. A mixture of two products is normally obtained, being the α and β glycofuranosyl or glycopyranosyl derivatives at the point of ring attachment. These can normally be separated by the aforementioned chromatographic methods.

After separation of the individual products, the glycosidic residues are deacylated in base, such as sodium methoxide in methanol, of ammonia in methanol. Further purification by high performance chromatography is usually indicated to obtain the highly purified product.

When the starting vitamin D (D₃ or D₂) carries two hydroxy groups (such as 1-hydroxy vitamin D₃, or 25-hydroxy vitamin D₃) one of these needs to be selectively protected with a protecting group which can be ultimately removed after the condensation , and before, during or after the deacylation of the glycosidic residues. The same is true if three or more hydroxy groups are present in the vitamin starting materials, and less than all of these require to be glycosylated.

The selective protection of hydroxy groups in the starting materials can be carried out by using standard protection and deprotection reactions, well known to those skilled in Organic Chemistry.

Because each of the hydroxyl groups on the vitamin D molecule have different reactivities either due to the fact that they are either primary (e.g. 26-OH), secondary (eg. 24-OH, 3β-OH, etc.) or tertiary (eg. 25-OH) hydroxyl functions, selectivity can be achieved. Furthermore, because of steric considerations the 3β-OH has different reactivity than the 1α-OH which is both a vicinyl hydroxyl function as well as sterically hindered by the exocylic C₁₉ methylene function on C₁₀. A good example of these reactivities is illustrated in Holick et al, Biochemistry: 10, 2799, 1971, where it is shown that the trimethylsilyl ether derivative of 1,25-(OH)₂-D₃ can be hydroxylated in HCl-MeOH under mild conditions to yield 3,25-disilyl ether, and 25-monosilyl ether derivatives of 1,25-(OH)₂-D₃. Furthermore, to obtain a 1,25-(OH)₂-D₃ whereby the 3 and 1 hydroxyls are protected, the 25-monosilyl ether derivative of 1,25-(OH)₂-D₃ can be acetylated to form the 1,25-(OH)₂-D₃-1,3-diacetyl-25-trimethyl silyl ether. Because the acetates are quite stable to acid hydrolyis, this derivative can be acid hydrolyzed to yield 1,3-diacetoxy-25-hydroxyvitamin D₃. An alternative approach would simply be to acetylate 1,25-(OH)₂-D₃ in acetic anhydride in pyridine at room temperature for 24 to 48 to yield 1,3-diacetoxy-25 hydroxyvitamin D₃.

For protecting the 25-hydroxyl group for 25-hydroxyvitamin D₃ the following can be done: 25-OH-D₃ can be completely acetylated in acetic anhydride and pyridine under refluxing conditions for 24 h. The 3-Ac can be selectively removed by saponification (KOH in 95% MeOH-water) at room temperature for 12 h.

Once the desired protected vitamin D derivative is prepared, the same is reacted with silver carbonate or other methods for coupling (as described e.g. by Igarashi, K., in "Advances in Carbohydrate Chemistry and Biochemistry," Vol 34, 243-283, or Warren, C.D. et al, Carbohydrate Research, 82: 71-83 (1980)), and the glycosidic or polyglycosidic residue as in Scheme I above, followed by deacylation, deprotection and purification. Among the starting vitamin D derivatives which are readily available, are, for example:
Vitamin D₃;
Vitamin D₂;
1-hydroxy-Vitamin D₃;
1-hydroxy-Vitamin D₂;
25-OH-Vitamin D₃;
25-OH-Vitamin D₂;
1,24-(OH)₂-Vitamin D₃;
1,25-dihydroxy-Vitamin D₃;
1,25-dihydroxy-Vitamin D₂;
24,25-dihydroxy-Vitamin D₃;
25,26-dihydroxy-Vitamin D₃;
24,25-dihydroxy-Vitamin D₂;
1,24,25-trihydroxy-Vitamin D₃;
1,25,26-trihydroxy-Vitamin D₃;
Some materials, such as 25,26-Vitamin D₂, 1,24,25-trihydroxy Vitamin D₂ or 1,25,26-trihydroxy Vitamin D₂ have not yet been fully identified in the art, but can nevertheless be used if synthetically prepared.

The acylated glycoside containing a leaving group at position C-1' of the first (or only) glycosidic ring can be prepared, for example, by the methods of Fletcher, H.G., Jr., "Methods in Carbohydrate Chemistry" 2: 228 (1963), or Bonner, W.A., Journal of Organic Chemistry 26: 908-911 (1961), or Lemieux, R.U., "Methods in Carbohydrate Chemistry", Vol. II, 221,222.

Oligosaccharide intermediates can be prepared, for example by the methods of Lemieux, R.U., J. of Amer. Chem. Soc. 97: 4063-4069 (1975); of Frechet, J.M.J., "Polymer-Supported Reactions in Organic Synthesis" (1980) 407-434, or Kennedy, J.F., "Carbohydrate Chemistry" 7: 496-585 (1975).

Commercially available sugars include (Pfanstiehl Laboratories, Inc.): Pentoses, such as: D-arabinose, L-arabinose, D-Lyxose, L-Lyxose, D-Ribose, D-Xylose, L-Xylose; Hexoses, such as: Dextroses, D-Fructose, D-Galactose, α-D-Glucose, β-D-Glucose, L-Glucose, Levulose, D-mannose, L-Mannose, L-Sorbose; Heptoses, such as: D-Glucoheptose, D-Mannoheptulose, Sedoheptulosan; Disaccharides, such as: Cellobiose, 3-O-β-D- Galactopyranosyl-D-arabinose, Gentiobiose, Lactoses, α-Lactulose, Maltose, α-Melibiose, Sucrose, Trehalose, Turanose Trisaccharides, such as : Melezitose, Raffinose, Tetrasaccharides, such as: Stachyose; Polysaccharides and derivatives, such as: Arabic Acid, Chitin, Chitosan, Dextrin, Cyclo-Dextrins, Glycogen, Inulin.

Alternatively, the whole synthetic sequence (protection, condensation and deprotection) can be carried out starting with a Δ⁵,⁷ steroidal diene which is a provitamin D. After glycosylation, the provitamin is ring-opened photochemically, and the resulting previtamin is thermally rearranged to yield glycosilated vitamin.

It is known (Napoli, J.L. and DeLuca, H.F., in "Burger's Medicinal Chemistry" 4th Ed., part II, page 728 ff) that the active form of vitamin D is 1,25-dihydroxyvitamin D₃. When 1,25-dihydroxy-vitamin D₃ glycoside is used in the treatment of hypocalcemic states, or the regulation of phosphorus and calcium metabolism in an animal, especially in a human, the endogenous glycosidase enzymes of the animal directly release the active form of the vitamin. On the other hand, when non-hydroxylated derivatives of the vitamin are used (such as, e.g., vitamin D₃ glycoside), enzymatic release of the hydroxylated vitamin is followed by hydroxylation in the liver and then in the kidney in order to form the active 1,25-dihydroxy vitamin.

The water-soluble glycosilated vitimin D conjugates of the present invention include hydrophilic derivatives of good water solubility to derivatives of excellent water-solubility. They can be used generally in any application where the use of vitamin D₃, vitamin D₂ or hydroxylated derivatives thereof has been called for in the prior art. The advantage of the conjugates of the invention resides in their water-solubility and thus their ease of administration in aqueous media such as, for example, saline or aqueous buffers. This allows the utilization of these conjugates in such devices as vitamin D releasing in-line pumps and intravenous dispensation. Other advantages include treatment of fat malabsorption syndromes, as well as release of the biologically active form of Vitamin D₃ in the gut, e.g. 1,25-(OH)₂-D₃ glycoside → gut → 1,25(OH)₂-D₃ → biological action.

The conjugates of the invention can be administered by any means that effect the regulation of calcium and phosphorus homeostasis and metabolism in animals, especially humans. For example, administration can be topical, parenteral, subcutaneous, intradermal, intravenous , intramuscular, or interperitoneal. Alteratively or concurrently, administration can be by the oral route. The dosage administered will be dependent upon the age, health and weight of the recipient, kind of concurrent treatment if any, frequency of treatment, and the nature of the effect desired. Generally, a dosage of active ingredient compounds will be from about 0.1 µg to 1 mg per kg of body weight. Normally, from 0.1 µg to 10 µg per kg per application, in one or more applications per therapy, is effective to obtain the desired result.

An additional unexpected property of the compounds of the invention is that some of them may demonstrate promotion of calcium absorption through the intestine without effecting calcium mobilization brought about by calcium release from bones. Calcium mobilization by bone release is a common feature of 1,25 dihydroxy-vitamin D₃. Its selective absence in some of the compounds of the invention has a beneficial therapeutic consequence by promoting an increase in serum calcium levels by stimulating intestinal calcium transport. It is disadvantageous for patients with severe bone disease to maintain serum calcium levels at the expense of mobilizing calcium from their already wasting bones.

The compounds can be employed in dosage forms such as tablets, capsules, powder packets or liquid solutions, suspensions or elixirs for oral administration, or sterile liquids for formulations such as solutions or suspensions for parenteral use. In such compositions, the active ingredient will ordinarily always be present in an amount of at least 1x10⁻⁶% by wt. based upon the total weight of a composition, and not more than 90% by wt. An inert pharmaceutically acceptable carrier is preferably used. Among such carriers are 95% ethanol, vegetable oils, propylene glycols and saline buffers.

Having now generally described this invention, a more complete understanding can be obtained by reference to certain examples, which are included herein for purposes of illustration only and are not intended to be limiting unless otherwise specified.

### Example 1

### Preparation of Vitamin D₃, 3β -glucoside.

In a 3-neck 100-ml round-bottom flask, equipped with dropping funnel and distillation head, was suspended 1.00 g (3.63 mmole) of dry silver carbonate, freshly prepared according to the procedure of Becker, Biochem. Biophys. Act: 100: 574-581 (1965), in 5 ml of dry benzene, in which was dissolved 147 mg (0.382 mmole) of vitamin D₃. The solution was brought to boiling. At that point, 647 mg (1.57 mmole) of tetra-O-acetyl -β- D-glucopyranosyl bromide, prepared according to the procedure of Lemieux, supra, dissolved in 25 ml of benzene, was added drop-by-drop. Benzene continued to distill and about 1/2 hour later more silver carbonate (approximately 1 g) was added to the reaction mixture. The reaction was followed by thin layer chromatography (20:80 v/v ethyl acetate/hexane). The minor product had an R_{f} of 0.24, and the major product had an R_{f} of 0.20. After two hours, the reaction mixture was cooled and then filtered through glass wool to remove the silver salt. The filtrate was dried over anhydrous sodium sulfate, and the benzene was evaporated under nitrogen. The resulting yellow oil was applied to a preparative µ-Porasil high-pressure liquid chromatographic column (dimensions, 8 mmx30cm; flow rate: 2 ml/min; solvent 15/85 v/v ethyl acetate/hexane). The major product, 9,10-secocholesta-5,7,10(19)-trien -3β- yl-2',3',4',6'-tetra-O-acetyl -β- D-glucopyranoside, with a retention time of 58 minutes, exhibited an absorbance maximum of 265 nm, and an absorbance minimum of 228 nm, characteristic of the triene chromophore in vitamin D. Its mass spectrum contains a peak for the parent molecular ion and at m/e 714, 2.5% (M⁺); peaks at 383, 5% (M-pyronium ion); 366, 28% (M-pyronium ion-water)⁺; 351, 18%; 331, 15% (pyronium ion)⁺; 271, 2.5%; 253, 14%; 169 100%; (C₈H₉O₄)⁺; 109,63% (C₆H₅O₂)⁺; and 60, 20% (methyl formate or acetic acid). A minor product 9,10-secocholesta-5,7,10(19)-triene -3β- yl-2',3',4',6',-tetra-O-acetyl -α- D-glucopyranoside with a retention time of 45 minutes, also exhibited an absorbance maximum at 265 nm and an absorbance minimum at 228 nm. Its mass spectrum exhibited a molecular ion of m/e 714.

The major product, having the retention time of 58 minutes, was then deacylated with sodium methoxide and methanol. A small piece of sodium metal was added to the compound dissolved in anhydrous methanol. After 1/2 hour the solution was neutralized with dilute acetic acid. The solution was dried under nitrogen and then applied to a reverse-phase high-pressure liquid chromatographic column (Radial Pak A column Waters Associates, dimensions 0.8 X 10 cm; flow rate 1 ml/min; solvent 98/2 v/v methanol/water). The product, 9,10-secocholesta-5,7,10(19)triene -3β- yl -β- D-glucopyranoside, had a retention time of 12.5 minutes and exhibited the UV spectrum, λₘₐₓ 265 nm, λₘᵢₙ 228 nm , typical of the vitamin D chromophore.

The vitamin D₃, 3β-glucoside vitamin D₃, 3α-glucoside and the vitamin D₃, 3β glucoside acetate were tested for biological activity. Male weanling rats from Holtzmann Company, Madison, Wisconsin, U.S.A., were fed a vitamin D deficient diet that was adequate in phosphorus and low in calcium (0.02%) for 3-1/2 weeks. Groups of five animals received orally either 4 µg, 1 µg, 0.5 µg, 0.25 µg of Vitamin D₃ -3β- glucoside, 1 µg Vitamin D₃ -3α- glucoside or 2 µg Vitamin D₃ -3β- glucoside acetate in 50 µl of 95% ethanol or vehicle alone. 24 hours later the animals were sacrificed and the small intestine and blood were collected. Intestinal calcium transport studies were performed by the everted gut sac technique, and blood was used for serum calcium determinations. The results are shown in the following table:

| Compound | I/O (inside Ca⁴⁵/outside Ca⁴⁵) ± S.D. | Serum Calcium ± S.D. |
|---|---|---|
| 95% ethanol | 1.6 ± 0.3 | 4.3 ± 0.2 |
| 4µg Vitamin D₃ 3β- glucoside | 3.9 ± 0.2 | 5.3 ± 0.2 |
| 1 µg do. | 3.6 ± 0.3 | |
| 0.5 µg do. | 2.5 ± 0.2 | |
| 0.25 µg do. | 2.0 ± 0.2 | |
| 1µg Vitamin D₃ 3α- glucoside | 2.0 ± 0.4 | |
| 2µg Vitamin D₃ 3β- glucoside acetate | 1.7 ± 0.2 | |

The data show that the vitamin D₃, 3β -glucoside is capable of stimulating intestinal calcium absorption, and bone calcium mobilization. The 3α glucoside is somewhat less active, while the 3β- acetate appears inactive.

## Claims (Claims for the following Contracting State(s): BE, CH, DE, FR, GB, LI, LU, NL, SE)

1. A synthetic compound which is biologically active in maintaining calcium and phosphorous metabolism in animals, of the formula I: 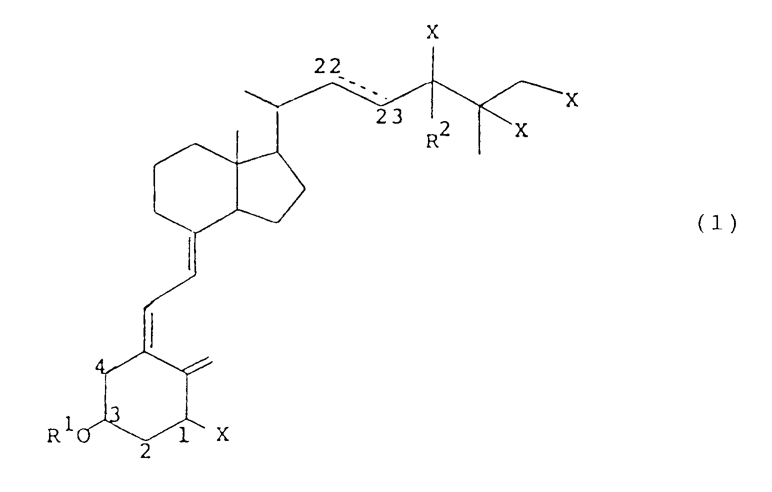 wherein
the bond between positions C-22 and C-23 is single or double;
the bond at position C-3 is β;
R² is hydrogen, methyl or ethyl;
X is selected from hydrogen and -OR¹, where R¹ is a glycosidic residue selected from glucose, mannose, galactose, gulose, allose, altrose, idose, talose, fructose, arabinose, xylose, sucrose, cellobiose, maltose, lactose, trehalose, gentiobiose, meliobiose, raffinose, gentianose, N-acetyl-D-galactosamine, N-acetyl-D-glucosamine, N-acetyl-D-mannosamine, N-acetylneuraminic acid, D-glucosamine, lyxosylamine and D-galactosamine.

2. The compound of Claim 1 wherein, when X at position C-1 is -OR¹ the bond at C-1 is α.

3. The compound of Claim 1 wherein the bond between C-22 and C-23 is single, and R² = H.

4. The compound of Claim 1 which is of formula 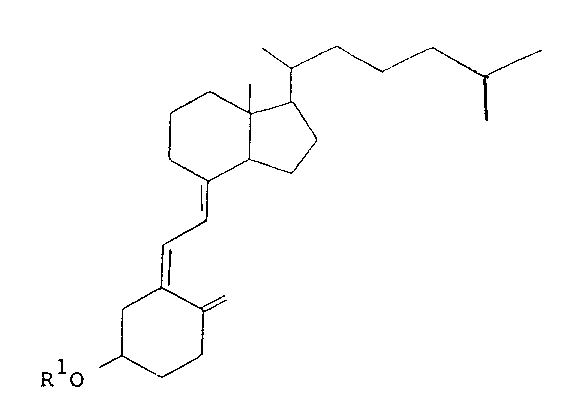 wherein R¹ is a glycosidic residue as defined in claim 1.

5. The compound of Claim 1 which is of formula 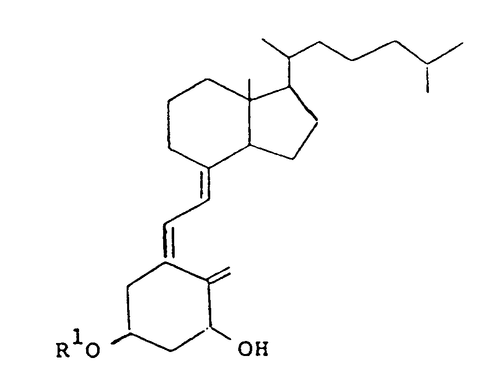 wherein R¹ is a glycosidic residue as defined in claim 1.

6. The compound of Claim 1 which is of formula 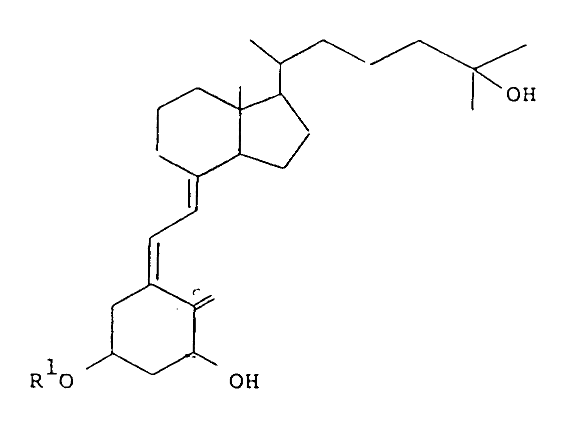 wherein R¹ is a glycosidic residue as defined in claim 1.

7. The compound of Claim 1 which is of formula 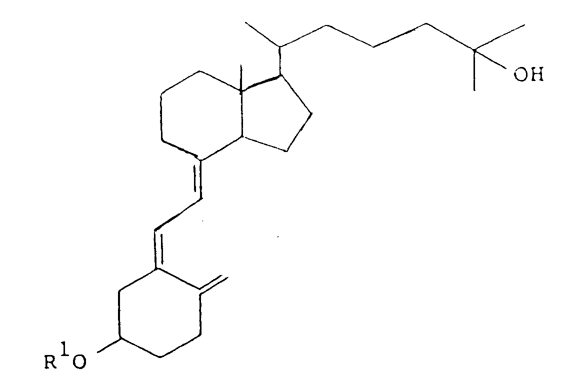 wherein R¹ is a glycosidic residue as defined in claim 1.

8. Composition for regulating calcium and phosphorous homeostasis in animals comprising a water soluble synthetic compound as defined in claims 1 to 7.

## Claims (Claims for the following Contracting State(s): AT)

1. A method for preparing a synthetic compound which is biologically active in maintaining calcium and phosphorous metabolism in animals of the formula I: 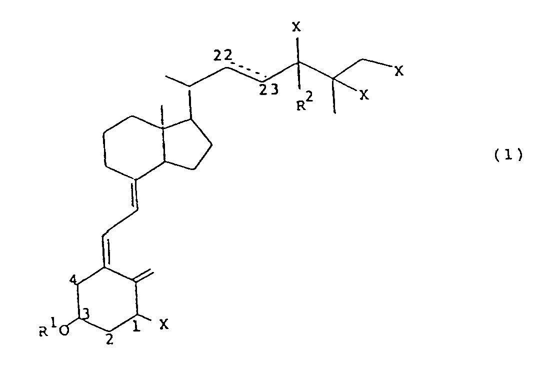 wherein
the bond between positions C-22 and C-23 is single or double;
the bond at position C-3 is β;
R² is hydrogen, methyl or ethyl;
X is selected from hydrogen and -OR¹, wherein R¹ is a glycosidic residue selected from glucose, mannose, galactose, gulose, allose, altrose, idose, talose, fructose, arabinose, xylose, sucrose, cellobiose, maltose, lactose, trehalose, gentiobiose, meliobiose, raffinose, gentianose, N-actyl-D-galactosamine, N-acetyl-D-glucosamine, N-acetyl-D-mannosamine, N-acetylneuraminic acid, D-glucosamine, lyxosylamine and D-galactosamine,
comprising the steps of
a) reacting in a manner known in the art a vitamin starting material selected from the group consisting of
Vitamin D₃;
Vitamin D₂;
1-hydroxy-Vitamin D₃;
1-hydroxy-Vitamin D₂;
25-OH-Vitamin D₃;
25-OH-Vitamin D₂;
1,24-(OH)₂-Vitamin D₃;
1,25-dihydroxy-Vitamin D₃;
1,25-dihydroxy-Vitamin D₂;
24,25-dihydroxy-Vitamin D₃;
25,26-dihydroxy-Vitamin D₃;
24,25-dihydroxy-Vitamin D₂;
1,24,25-trihydroxy-Vitamin D₃;
1,25,26-trihydroxy-Vitamin D₃;
with a fully acylated glycoside selected from glucose, mannose, galactose, gulose, allose, altrose, idose, talose, fructose, arabinose, xylose, sucrose, cellobiose, maltose, lactose, trehalose, gentiobiose, meliobiose, raffinose, gentianose, N-acetyl-D-galactosamine, N-acetyl-D-glucosamine, N-acetyl-D-mannosamine, N-acetylneuraminic acid, D-glucosamine, lyxosylamine and D-galactosamine, each containing an appropriate leaving group at position C-1' of the terminal ring or on the single ring, respectively in the presence of silver carbonate and in an inert nonpolar solvent, and
b) deacylating and purifying the resulting product in a manner known in the art to obtain said synthetic compound having formula I.

2. The method of claim 1, wherein, when X at position C-1 is -OR¹ the bond at C-1 is α.

3. The method of Claim 1, wherein the bond between C-22 and C-23 is single, and R² = H.

4. The method of Claim 1 in which the compound is of formula 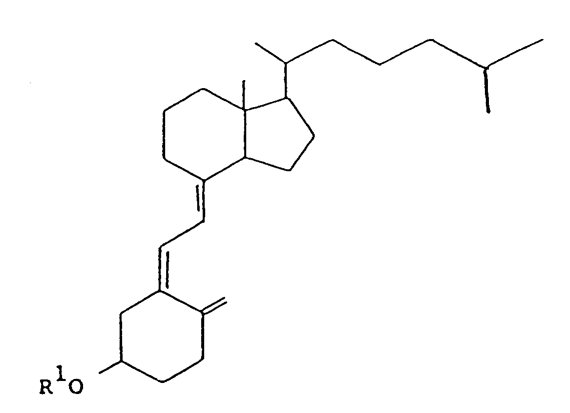 wherein R¹ is a glycosidic residue as defined in claim 1.

5. The method of Claim 1 in which the compound is of formula 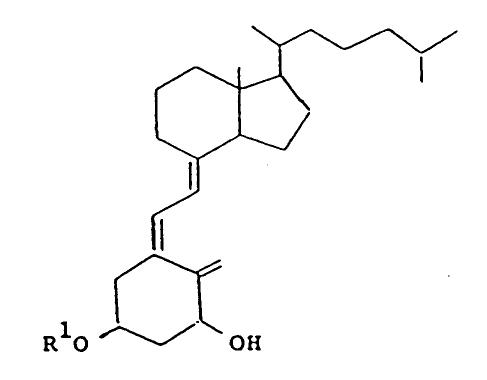 wherein R¹ is a glycosidic residue as defined in claim 1.

6. The method of Claim 1 in which the compound is of formula 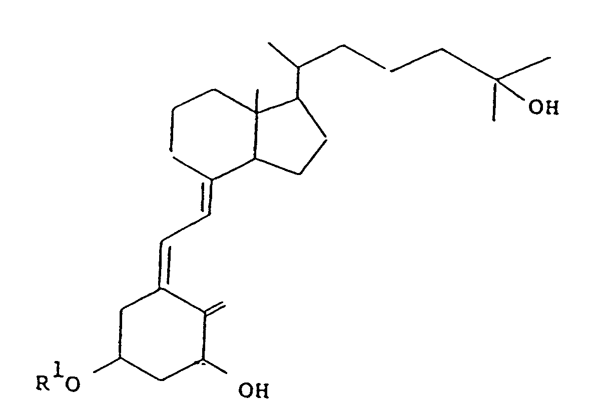 wherein R¹ is a glycosidic residue as defined in claim 1.

7. The method of Claim 1 in which the compound is of formula 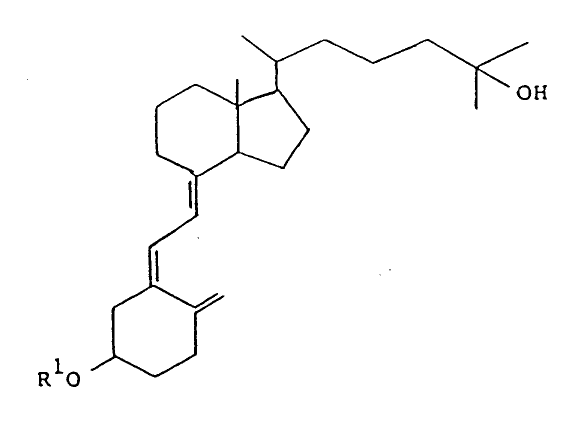 wherein R¹ is a glycosidic residue as defined in claim 1.

## Patentansprüche (Patentansprüche für folgende(n) Vertragsstaat(en): BE, CH, DE, FR, GB, LI, LU, NL, SE)

1. Synthetische Verbindung, die biologisch wirksam ist in der Beibehaltung des Calcium- und Phosphor-Metabolismus in Tieren, mit der Formel I: 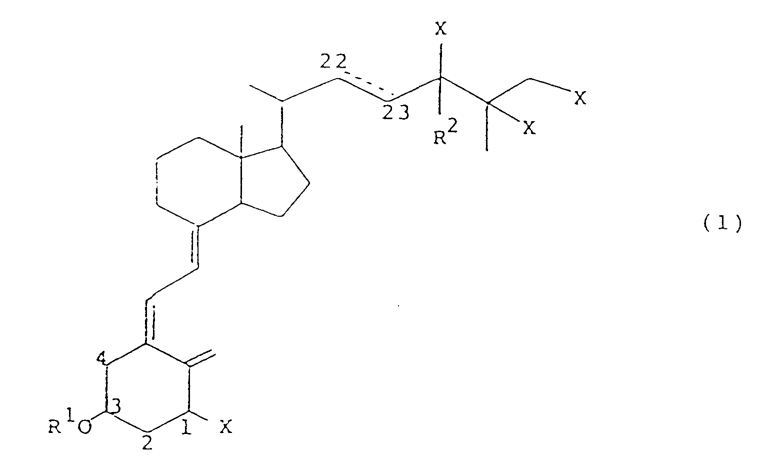 worin die Bindung zwischen Positionen C-22 und C-23 eine Einzel- oder Doppelbindung ist; die Bindung an Position C-3 β ist; R² Wasserstoff, Methyl oder Ethyl ist; X ausgewählt ist aus Wasserstoff und -OR¹, worin R¹ ein glycosidischer Rest ist, ausgewählt aus Glucose, Mannose, Galactose, Gulose, Allose, Altrose, Idose, Talose, Fructose, Arabinose, Xylose, Saccharose, Cellobiose, Maltose, Lactose, Trehalose, Gentiobiose, Melibiose, Raffinose, Gentianose, N-Acetyl-D-galactosamin, N-Acetyl-D-glucosamin, N-Acetyl-D-mannosamin, N-Acetylneuraminsäure, D-Glucosamin, Lyxosylamin und D-Galactosamin.

2. Verbindung nach Anspruch 1, dadurch gekennzeichnet, daß, wenn X an Position C-1 -OR¹ ist, die Bindung an C-1 α ist.

3. Verbindung nach Anspruch 1, dadurch gekennzeichnet, daß die Bindung zwischen C-22 und C-23 eine Einfachbindung ist und R² = H ist.

4. Verbindung nach Anspruch 1 mit der Formel 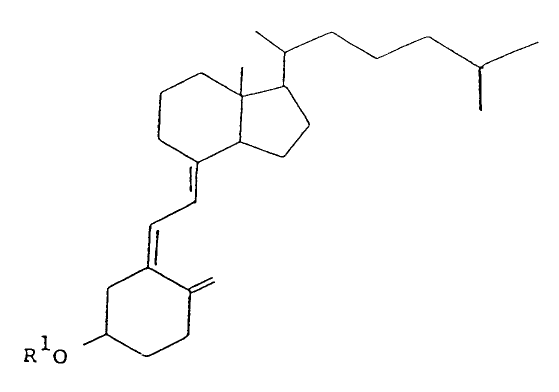 worin R¹ ein glycosidischer Rest, wie in Anspruch 1 definiert, ist.

5. Verbindung nach Anspruch 1 mit der Formel 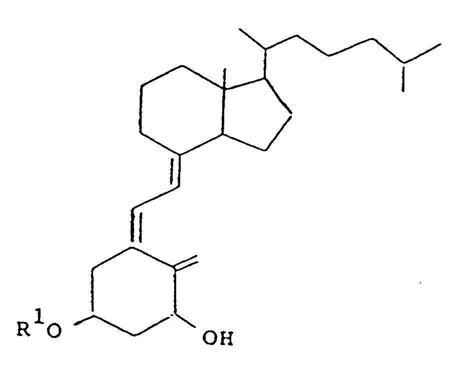 worin R¹ ein glycosidischer Rest, wie in Anspruch 1 definiert, ist.

6. Verbindung nach Anspruch 1 mit der Formel 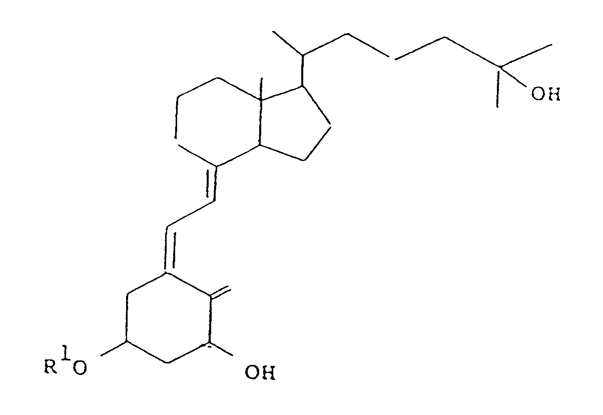 worin R¹ ein glycosidischer Rest, wie in Anspruch 1 definiert, ist.

7. Verbindung nach Anspruch 1 mit der Formel 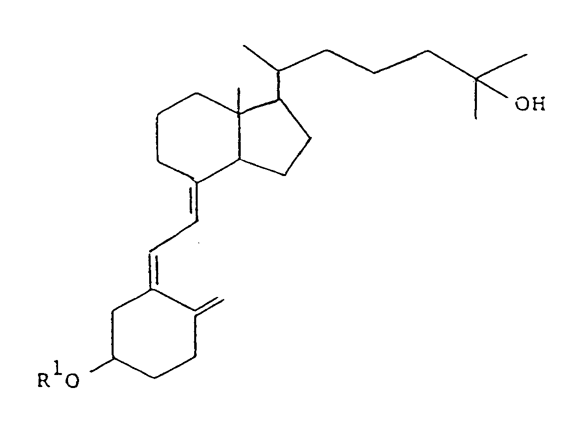 worin R¹ ein glycosidischer Rest, wie in Anspruch 1 definiert, ist.

8. Zusammensetzung zur Regulierung der Calcium- und Phosphor-Homöostase in Tieren, umfassend eine wasserlösliche, synthetische Verbindung, wie in Ansprüchen 1 bis 7 definiert.

## Patentansprüche (Patentansprüche für folgende(n) Vertragsstaat(en): AT)

1. Verfahren zur Herstellung einer synthetischen Verbindung, die biologisch wirksam ist in der Beibehaltung des Calcium- und Phosphor-Metabolismus in Tieren, mit der Formel I: 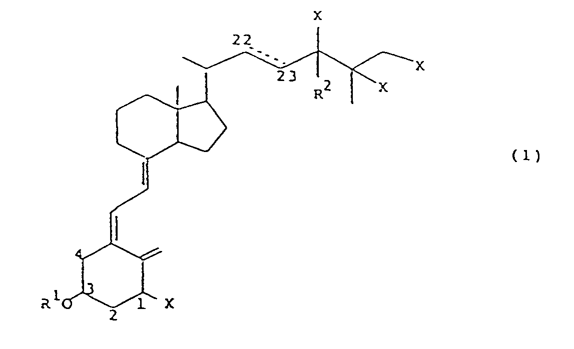 worin die Bindung zwischen Positionen C-22 und C-23 eine Einzel- oder Doppelbindung ist; die Bindung an Position C-3 β ist; R² Wasserstoff, Methyl oder Ethyl ist; X ausgewählt ist aus Wasserstoff und -OR¹, worin R¹ ein glycosidischer Rest ist, ausgewählt aus Glucose, Mannose, Galactose, Gulose, Allose, Altrose, Idose, Talose, Fructose, Arabinose, Xylose, Saccharose, Cellobiose, Maltose, Lactose, Trehalose, Gentiobiose, Melibiose, Raffinose, Gentianose, N-Acetyl-D-galactosamin, N-Acetyl-D-glucosamin, N-Acetyl-D-mannosamin, N-Acetylneuraminsäure, D-Glucosamin, Lyxosylamin und D-Galactosamin,
umfassend die Schritte
a) das Umsetzen, in einer auf dem Fachgebiet bekannten Art und Weise, eines Vitamin-Ausgangsmaterials, ausgewählt aus der Gruppe bestehend aus
Vitamin D₃; Vitamin D₂; 1-Hydroxy-Vitamin D₃; 1-Hydroxy-Vitamin D₂; 25-OH-Vitamin D₃; 25-OH-Vitamin D₂; 1,24-(OH)₂-Vitamin D₃; 1,25-Dihydroxy-Vitamin D₃; 1,25-Dihydroxy-Vitamin D₂; 24,25-Dihydroxy-Vitamin D₃; 25,26-Dihydroxy-Vitamin D₃; 24,25-Dihydroxy-Vitamin D₂; 1,24,25-Trihydroxy-Vitamin D₃; 1,25,26-Trihydroxy-Vitamin D₃;
mit einem völlig acylierten Glycosid, ausgewählt aus Glucose, Mannose, Galactose, Gulose, Allose, Altrose, Idose, Talose, Fructose, Arabinose, Xylose, Saccharose, Cellobiose, Maltose, Lactose, Trehalose, Gentiobiose, Melibiose, Raffinose, Gentianose, N-Acetyl-D-galactosamin, N-Acetyl-D-glucosamin, N-Acetyl-D-mannosamin, N-Acetylneuraminsäure, D-Glucosamin, Lyxosylamin und D-Galactosamin, wobei jedes eine geeignete Abgangsgruppe an der Position C-1' des terminalen Rings beziehungsweise des einzigen Rings enthält, in der Gegenwart von Silbercarbonat und in einem inerten, unpolaren Löungsmittel, und
b) das Entacylieren und das Reinigen des resultierenden Produkts in einer auf dem Fachgebiet bekannten Art und Weise, um die synthetische Verbindung mit der Formel I zu erhalten.

2. Verfahren nach Anspruch 1, dadurch gekennzeichnet, daß, wenn X an Position C-1 -OR¹ ist, die Bindung an C-1 α ist.

3. Verfahren nach Anspruch 1, dadurch gekennzeichnet, daβ die Bindung zwischen C-22 und C-23 eine Einfachbindung ist und R² = H ist.

4. Verfahren nach Anspruch 1, dadurch gekennzeichnet, daβ die Verbindung die Formel 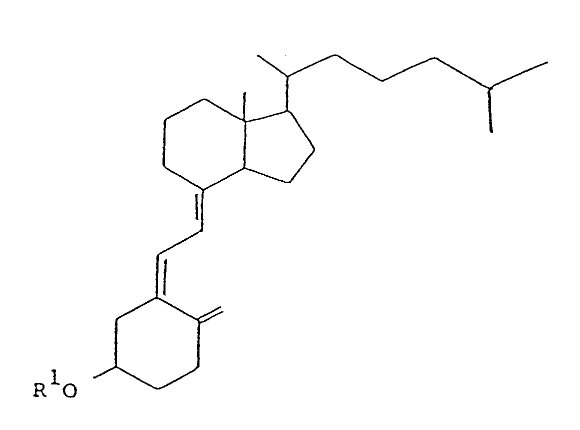 hat, worin R¹ ein glycosidischer Rest, wie in Anspruch 1 definiert, ist.

5. Verfahren nach Anspruch 1, dadurch gekennzeichnet, daß die Verbindung die Formel 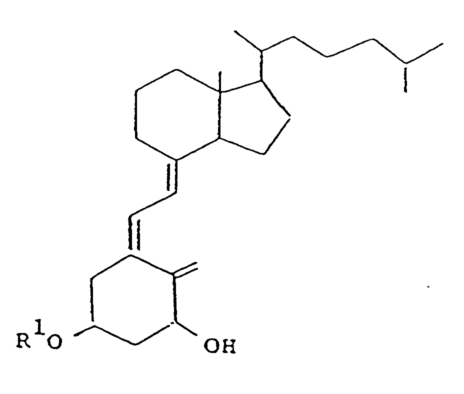 hat, worin R¹ ein glycosidischer Rest, wie in Anspruch 1 definiert, ist.

6. Verfahren nach Anspruch 1, dadurch gekennzeichnet, daß die Verbindung die Formel 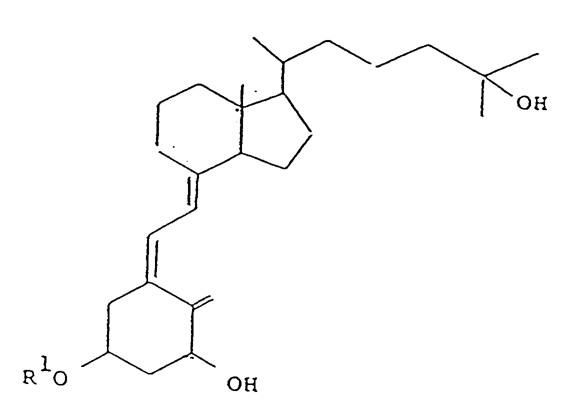 hat worin R¹ ein glycosidischer Rest, wie in Anspruch 1 definiert, ist.

7. Verfahren nach Anspruch 1, dadurch gekennzeichnet, daß die Verbindung die Formel 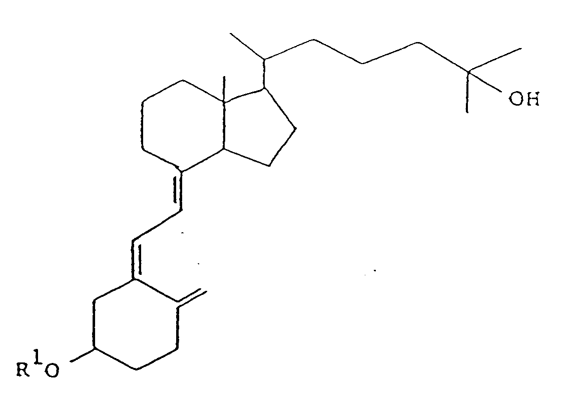 hat, worin R¹ ein glycosidischer Rest, wie in Anspruch 1 definiert, ist.

## Revendications (Revendications pour l'(les) Etat(s) contractant(s) suivant(s): BE, CH, DE, FR, GB, LI, LU, NL, SE)

1. Un composé synthétique biologiquement actif pour le maintien du métabolisme calcique et phosphoreux chez les animaux, de formule I : 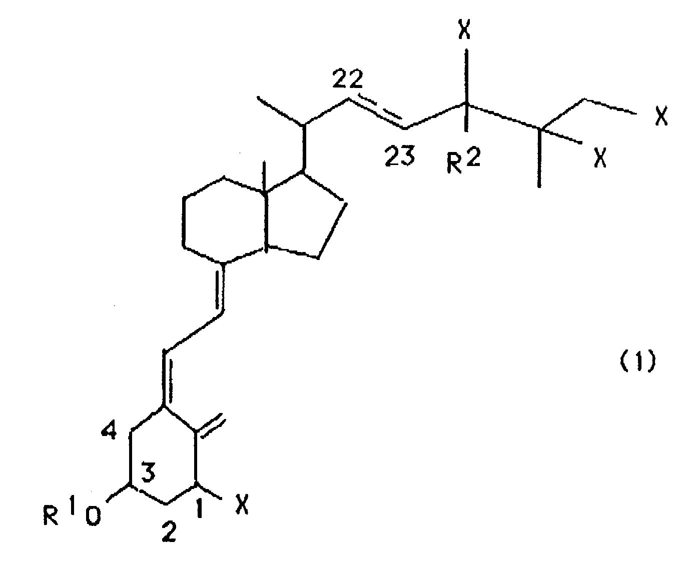 dans laquelle
la liaison entre les positions C-22 et C-23 est simple ou double;
la liaison à la position C-3 est de type β;
R² est l'hydrogène, le radical méthyl ou éthyl;
X est choisi parmi l'hydrogène et -OR¹, dans lequel R¹ est un résidu glycosidique choisie parmi le glucose, le mannose, le galactose, le gulose, l'allose, l'altrose, l'idose, le talose, le fructose, l'arabinose, le xylose, le sucrose, le cellobiose, le maltose, le lactose, le trehalose, le gentiobiose, le meliobiose, le raffinose, le gentianose, la N-acétyl-D-galactosamine, la N-acétyl-D-glucosamine, la N-acétyl-D-mannosamine, l'acide N-acétylneuraminique, la D-glucosamine, la lyxosylamine et la D-galactosamine.

2. Composé selon la revendication 1, dans lequel X, à la position C-1 est -OR¹ et la liaison en C-1 est de type α.

3. Composé selon la revendication 1, dans lequel la liaison entre C-22 et C-23 est simple, et R² = H.

4. Composé selon la revendication 1, de la formule : 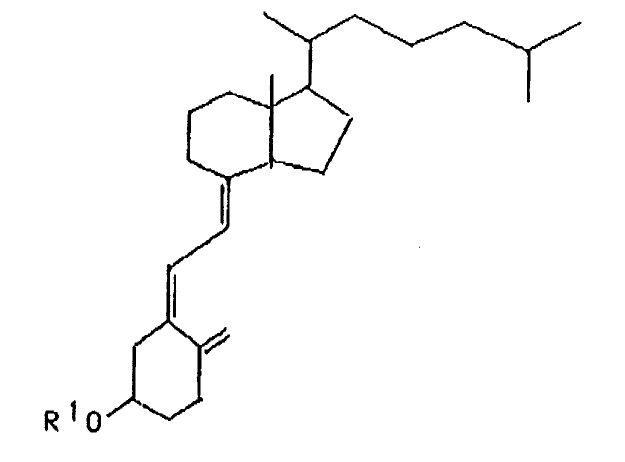 dans lequel R¹ est le résidu glycosidique_{,} défini dans la revendication 1.

5. Composé selon la revendication 1, de la formule : 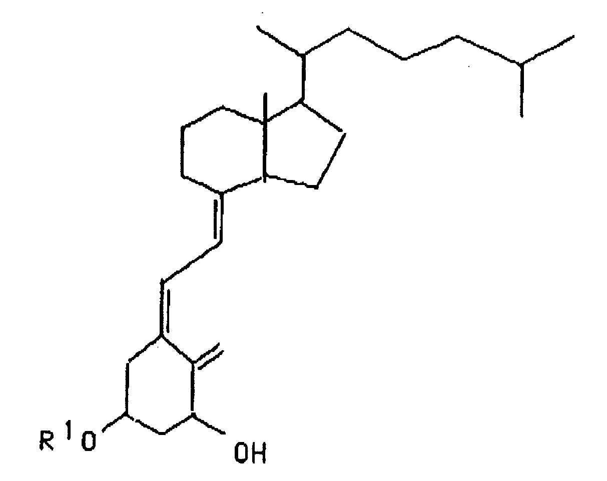 dans lequel R¹ est le résidu glycosidique, défini dans la revendication 1.

6. Composé selon la revendication 1, de la formule : 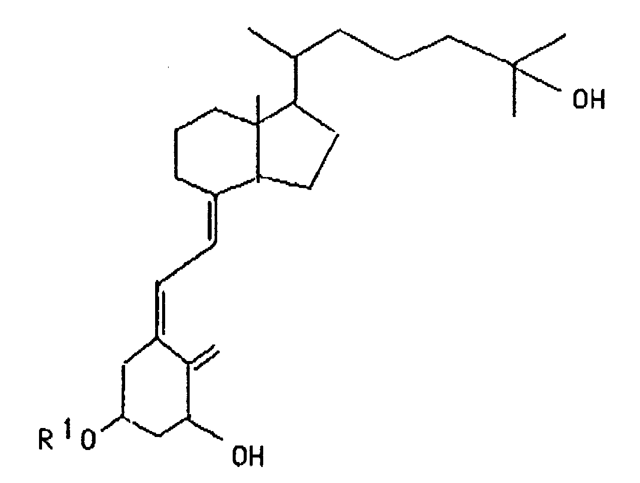 dans lequel R¹ est le résidu glycosidique, défini dans la revendication 1.

7. Composé selon la revendication 1, de la formule : 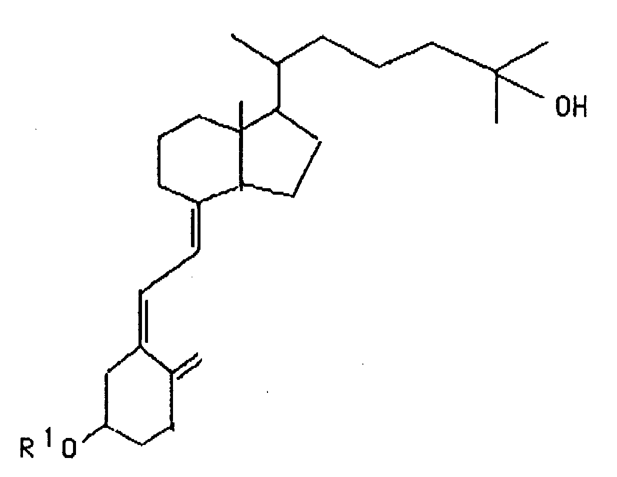 dans lequel R¹ est le résidu glycosidique, défini dans la revendication 1.

8. Composition pour réguler l'homéostase calcique et phosphoreuse chez les animaux, comprenant un composé synthétique soluble dans l'eau, tel que défini dans les revendications 1 à 7.

## Revendications (Revendications pour l'(les) Etat(s) contractant(s) suivant(s): AT)

1. Un procédé de préparation d'un composé synthétique biologiquement actif pour le maintien du métabolisme calcique et phosphoreux chez les animaux, de formule I : 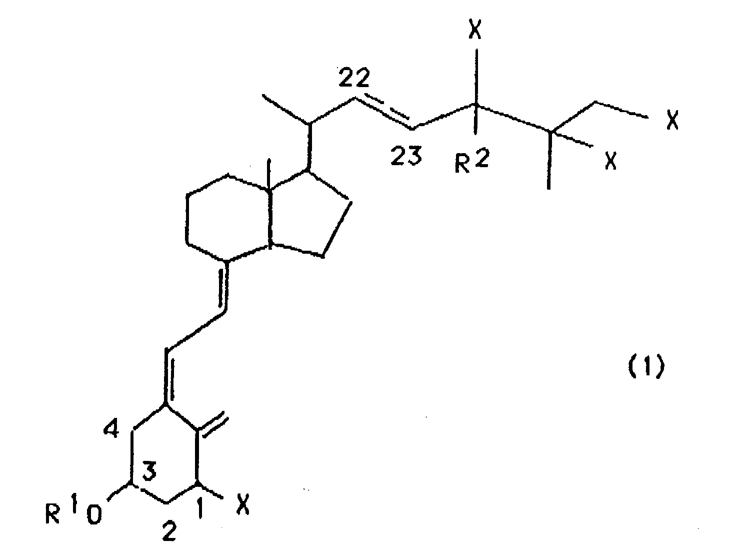 dans laquelle
la liaison entre les positions C-22 et C-23 est simple ou double;
la liaison à la position C-3 est de type β;
R² est l'hydrogène, le radical méthyl ou éthyl;
X est choisi parmi l'hydrogène et -OR¹, dans lequel R¹ est un résidu glycosidique choisi parmi le glucose, le mannose, le galactose, le gulose, l'allose, l'altrose, l'idose, le talose, le fructose, l'arabinose, le xylose, le sucrose, le cellobiose, le maltose, le lactose, le trehalose, le gentiobiose, le meliobiose, le raffinose, le gentianose, la N-acétyl-D-galactosamine, la N-acétyl-D-glucosamine, la N-acétyl-D-mannosamine, l'acide N-acétylneuraminique, la D-glucosamine, la lyxosylamine et la D-galactosamine, comprenant les étapes suivantes :
a) reaction de maniere connue, du type d'un materiau initiateur de vitamine, choisi dans le groupe composé de :
Vitamine D₃;
vitamine D₂;
1-hydroxy-vitamine D₃;
1-hydroxy-vitamine D₂;
25-OH-vitamine D₃;
25-OH-vitamine D₂;
1,24-(OH)₂-vitamine D₃;
1,25-dihydroxy-vitamine D₃;
1,25-dihydroxy-vitamine D₂;
24,25-dihydroxy-vitamine D₃;
25,26-dihydroxy-vitamine D₃;
24,25-dihydroxy-vitamine D₂;
1,24,25-trihydroxy-vitamine D₃;
1,25,26-trdihydroxy-vitamine D₃;
avec un glycoside entièrement acylaté, choisi parmi le glucose, le mannose, le galactose, le gulose, l'allose, l'altrose, l'idose, le talose, le fructose, l'arabinose, le xylose, le sucrose, le cellobiose, le maltose, le lactose, le trehalose, le gentiobiose, le meliobiose, le raffinose, le gentianose, la N-acétyl-D-galactosamine, la N-acétyl-D-glucosamine, la N-acétyl-D-mannosamine, l'acide N-acétylneuraminique, la D-glucosamine, la lyxosylamine et la D-galactosamine, chacun contenant un groupe donneur approprié en position C-1' de l'anneau terminal ou de l'anneau simple, respectivement en présence de carbonate d'argent et dans un solvant monopolaire inerte, et
b) désacylation de purification du produit résultant de manière connue, de façon à obtenir ledit composé synthétique de formule (I).

2. Composé selon la revendication 1, dans lequel X, à la position C-1 est -OR¹ et la liaison en C-1 est de type α.

3. Composé selon la revendication 1, dans lequel la liaison entre C-22 et C-23 est simple, et R² = H.

4. Composé selon la revendication 1, de la formule : 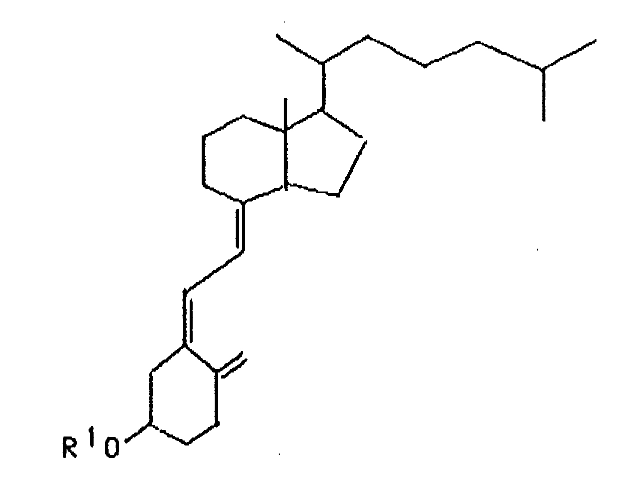 dans lequel R¹ est le résidu glycosidique, défini dans la revendication 1.

5. Composé selon la revendication 1, de la formule : 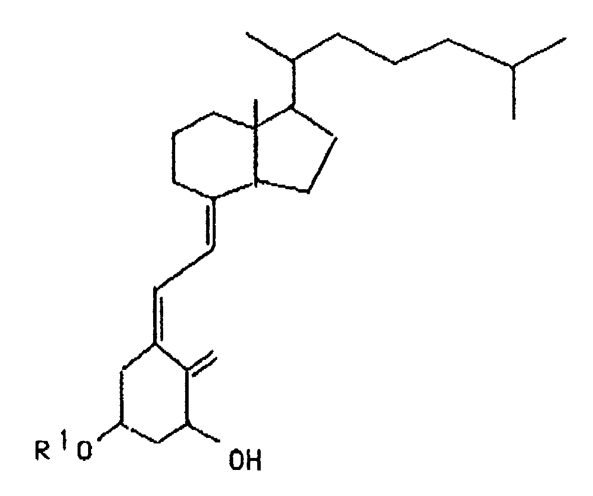 dans lequel R¹ est le résidu glycosidique, défini dans la revendication 1.

6. Composé selon la revendication 1, de la formule : 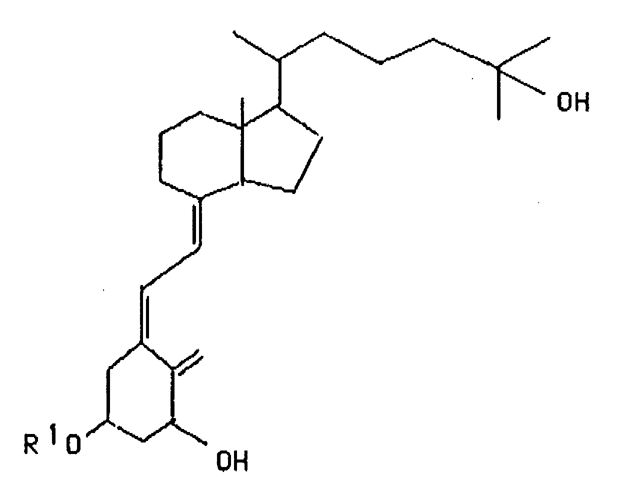 dans lequel R¹ est le résidu glycosidique, défini dans la revendication 1.

7. Composé selon la revendication 1, de la formule : 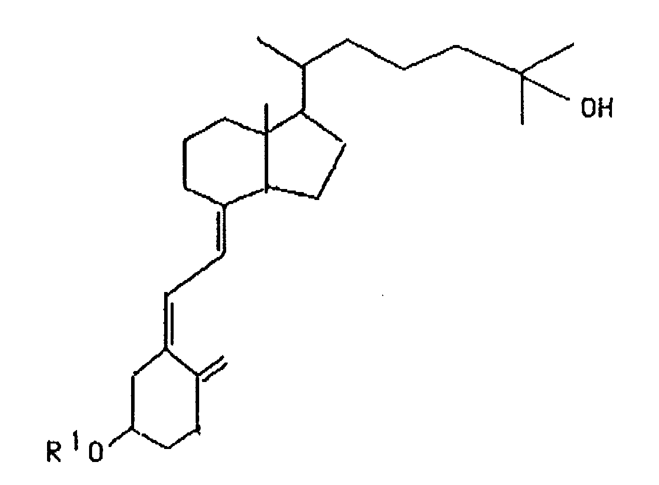 dans lequel R¹ est le résidu glycosidique, défini dans la revendication 1.
